# EUROPÄISCHE PATENTSCHRIFT

(11) **EP 0 869 182 B1**
(45) Veröffentlichungstag und Bekanntmachung des Hinweises auf die Patenterteilung: **25.07.2007**
(21) Anmeldenummer: 98101772.6
(22) Anmeldetag: 07.08.1992
(51) Int. Cl.: C12N 15/82

(54) **Pflanzen, die für ein Deacetylasegen transgen sind**
Plants transgenic for a deacetylase gene
Des plants transgénique pour un gène codant pour une déacétylase

(30) Priorität: 09.08.1991 DE 4126414
(43) Veröffentlichungstag der Anmeldung: 07.10.1998
(62) Teilanmeldung aus: 92113465.6
(73) Patentinhaber: Bayer CropScience AG, 40789 Monheim (DE)
(72) Erfinder: Broer, Inge, Dr., 18184 Roggentin (DE); Hilleman, Doris, 16278 Schönermark (DE); Pühler, Alfred, Prof. Dr., 33793 Bielefeld (DE); Wohlleben, Wolfgang, Prof. Dr., 72074 Tübingen (DE); Donn, Günter, Dr., 65619 Hofheim (DE); Müllner, Stefan, Dr., 65239 Hofheim (DE); Bartsch, Klaus, Dr., 61462 Königstein (DE)

(56) Entgegenhaltungen:
- EP-A- 0 257 542
- EP-A- 0 329 308
- WO-A-90/08828
- WO-A-91/03561
- JOURNAL OF GENERAL MICROBIOLOGY, Bd. 137, Nr. 2, Februar 1991, Seiten 351-359, XP002071458
- STRAUCH, E., ET AL.: "Cloning of a phosphinothricin N-acetyltransferase gene from Strpetomyces viridochromogenes Tü494 and its expression in Streptomyces lividans and Escherichia coli" GENE, Bd. 63, 1988, Seiten 65-74, XP002071459
- WOHLLEBEN, W., ET AL.: "Nucleotide sequence of the phosphinothricin N-acetyltransferase gene from Streptomyces viridochromogenes Tü494 and its expression in Nicotiana tabacum" GENE, Bd. 70, 1988, Seiten 25-37, XP002071460 -& WOHLLEBEN, W., ET AL.: EMBL SEQUENCE DATABASE, ACCESSION NO. M22827, 6.Juli 1989, XP002071461
- BIOLOGICAL ABSTRACTS, vol. 64, Philadelphia, PA, US; abstract no. 14329, CRABEEL, M., ET AL.: "Studies on the bipolar argECBH operon of E.coli: Characterization of restriction endonuclease fragments obtained from lambdadargeECBH transducing phages and a ColE1 argECBH plasmid" XP002071611 & MOL GEN GENET, Bd. 151, Nr. 2, 1977, Seiten 161-168,
- WOHLLEBEN, W., ET AL.: "Identification and characterization of phosphinothricin-tripeptide biosynthetic genes in Streptomyces viridochromogenes" GENE. PAPERS PRESENTED AT THE EIGHTH INTERNATIONAL SYMPOSIUM ON BIOLOGY OF ASCOMYCETES, UNIVERSITY OF WISCONSIN, MADISON, WI (USA), 11-16 AUGUST 1991, Bd. 115, Seiten 127-132, XP002071462
- NUSSBAUMER B.: EMBL SEQUENCE DATABASE, ACCESSION NO. X65195, 10.April 1992, XP002071463
- RAIBAUD, A., ET AL.: "Nucleotide sequence anlaysis reveals linked N-acteyl hydrolase, thioesterase, transport, and regulatory genes encoded by the bialaphos gene cluster of Streptomyces hygroscopicus" JOURNAL OF BACTERIOLOGY, Bd. 173, Nr. 14, Juli 1991, Seiten 4454-4463, XP002071464
- CHEMICAL ABSTRACTS, vol. 98, no. 9, 1983 Columbus, Ohio, US; abstract no. 66421, PIETTE, J., ET AL.: "The regulatory region of the divergent argECBH operon in Escherichia coli K-12" XP002071612 & NUCLEIC ACIDS RESEARCH, Bd. 10, Nr. 24, 1982, Seiten 8031-8048,

## Beschreibung

Die Erfindung betrifft Deacetylasegene, zur Erzeugung transgener Pflanzen unter Einsatz gewebespezifischer Promotoren, In diesen Pflanzen kann die Entwicklung bestimmter Pflanzenteile gezielt verhindert werden.

Phosphinothricin (PTC, 2-Amino-4-methylphosphinobuttersäure) ist ein Glutaminsynthetase(GS)-Inhibitor. PTC ist ein "Baustein" des Antibiotikums Phosphinothricyl-Alanyl-Alanin. Dieses Tripeptid (PTT) ist aktiv gegen Gram-positive und Gram-negative Bakterien und auch gegen den Pilz Botrytis cinerea. PTT wird von dem Stamm Streptomyces viridochromogenes Tü494 produziert, der bei der Deutschen Sammlung für Mikroorganismen unter den Nummern DSM 40736 und DSM 4112 hinterlegt und erhältlich ist.

Aus der Deutschen Patentschrift 2 717 440 ist bekannt, daß PTC als Totalherbizid wirkt. In der veröffentlichten Anmeldung (EP-A-0257542) ist beschrieben, wie man mit Hilfe eines Phosphinothricin-N-Acetyltansferase(pat)-Gens Herbizid-resistente Pflanzen herstellt. Die von dem pat-Gen kodierte Phosphinothricin-N-Acetyltransferase modifiziert das intrazellular auftretende PTC und detoxifiziert das Herbizid.

Raibaud et al. (Journal of Bacteriology, 173, 4454-4463, 1991) beschreiben, dass das Gen-Cluster der antibiotischen Bialaphos Produktion (bap) aus Streptomyces hygroscopicus für ein Genprodukt kodiert, welches Ähnlichkeiten zu Lipasen aufweist. Es gibt Hinweise darauf, dass es sich bei diesem Genprodukt um eine Acetylhydrolase (bah) handelt, welche die N-Acetyl-Gruppe, die sich an der demethylierten Form des PTC befindet, durch eine PTC-Acetyltransferase oder eine für demethyliertes PTC spezifische Acetyltransferase, die durch das Bialaphos-Resistenzegen (bar) kodiert wird, entfernt.

In WO91/03561 wird die Verwendung bakteriogener (*Streptomyces griseolus*) Cytochrom P450 Enzyme zur Erzeugung transgener Pflanzen beschrieben, wobei die so erhaltenen Pflanzen (a) durch enzymatische Inaktivierung herbizid wirkender Moleküle aus der Klasse der Sulfonylharnstoffe Resistenzen gegenüber diesen Herbiziden ausprägen können, oder aber (b) durch die Konvertierung nicht toxischer Substanzen in toxische männliche Sterilität ausprägen können.

Die vorliegende Erfindung beschreibt nun Deacetylasegene (dea), deren Expressionsprodukte intrazellulär N-Acetyl-Phosphinothricin (N-Ac-PTC) bzw. N-Ac-PTT deacerylieren können und so wieder antibiotisch aktiv machen.

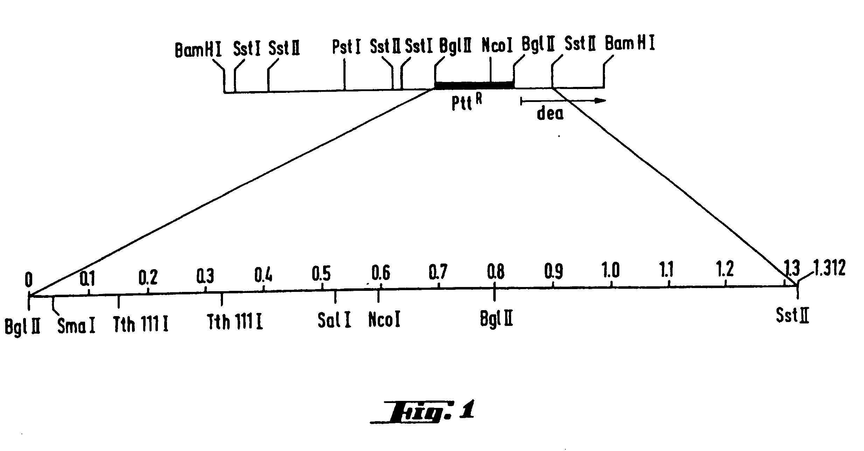

Ein erfindungsgemäßes N-Acetyl-Phosphinothricin-Tripeptid-Deacetylasegen läßt sich aus S. viridochromogenes Tü494 isolieren. EP0257542 beschreibt ein Resistenzgen gegen PTC, das aus *Streptomyces viridochromogenes* erhalten worden ist, und welches sich zur Herstellung PTC-resistenter Pflanzen eignet. Auf dem bereits bekannten 4.0-kb BamHl-Fragment (EP-A-0 257 542) liegt stromabwärts vom pat-Gen das dea-Gen. Dieses Gen liegt auf einem BgIII-BamHI-Fragment und ist durch die Sequenz genau bestimmt (Fig. 1 und Tab. 1). Die Proteinsequenz ist durch die DNA-Sequenz definiert. Als Translationsstartkodon dient ein ATG-Kodon, das in Bakterien und in Pflanzen erkannt wird; die Shine-Dalgarno-Sequenz ist durch Unterstreichen hervorgehoben. Dieses Gen kodiert in der PTT-Biosynthese den letzten Schritt, die Deacerylierung von inaktivem N-Acetyl-Phosphinothricin-Tripeptid zum aktiven PTT.

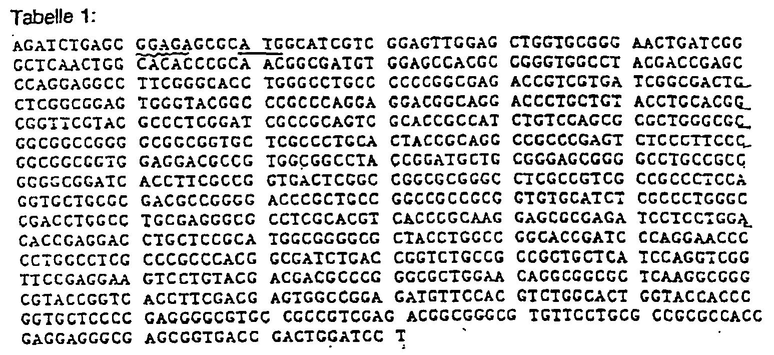

Von vielen Enzymen ist bekannt, daß ihre Spezifität nicht auf ein Substrat begrenzt ist So dient die vom pat-Gen kodierte Phosphinothricin-N-Acetyltransferase in der PTT-Biosynthese eigentlich zur Acetylierung von Desmethyl-PTC und kann aufgrund ihrer Unspezifität zur Detoxifizierung von PTC verwendet werden. Durch Überexpression des dea-Gens (mit Hilfe geeigneter Promotoren oder durch klonierung auf high-copy-Vektoren) kann eine nicht hinreichend spezifische N-Autyl-PTT-Deacetylase nun zur Aktivierung von N-Acetyl-Phosphinothricin eingesetzt werden.

Ein weiteres dea-Gen läßt sich aus E. coli gewinnen. Es wurde nämlich gefunden, daß sich in E. coli - im Gegensatz zu anderen Bakterien (z. B. Rhizobien und Streptomyceten) - nach Klonierung des pat-Gens in geeignete Expressionsvektoren (Strauch et al., Gene, 63, 65-74, 1988; Wohlleben et al., Gene, 70, 25-37, 1988) im sogenannten PAT-Assay (Doktorarbeit Inge Broer, Fakultät für Biologie der Universität Bielefeld, Expression des Phosphinthricin-N-Acetyltransferase-Gens aus Streptomyces viridochromogenes in Nicotiana tabacum, S. 42-43, 1989) keine Aktivität nachweisen läßt. Außerdem ist das pat-Gen in niedriger Kopienzahl in E. coli nicht in der Lage, PTT-Resistenz zu verleihen. da die endogene Deacetylase die Wirkung der Phosphinothricin-N-Acetyttransferase aufhebt. Schließlich kann diese Deacerylase-Aktivität durch die effektive Hemmung der GS-Aktivität nach Zugabe von N-Acetyl-Phosphinothricin direkt nachgewiesen werden. N-Ac-PTC wird durch die Deacetylase zu PTC umgesetzt, das dann in bekannter Weise die GS hemmt, was sich im γ-Glutamyl-Transferase-Assay (Bender et al., J. Bacteriol. 129, 1001-1009, 1977) messen läßt. Dies liegt an einer endogenen Deacetylase-Aktivität von E. coli.

Diese Aktivität sollte nicht in der argE-Mutante, die literaturbekannt ist, zu finden sein (Baumberg. Molec. Gen. Genetics 106, 162-173, 1970). Weitere E. coli Deacetylase-Mutanten sind leicht selektionierbar: Nach klassischer (Delic et al., Mut. Res. 9, 167-182, 1970; Drake und Baltz, Ann. Rev. Biochem. 45,11-38,1976) bzw. Tn5-Mutagenese (Kleckner, Ann. Rev. Genet. 15, 341-404, 1981) lassen sich auf mit PTT supplementiertem Minimalmedium solche Mutanten dadurch erkennen, daß nur sie nach Transformation mit einem in einen Niedrigkopienzahlvektor klonierten pat-Gen wachsen Können.

Damit läßt sich das Deacetylasegen aus E. coli durch Anlegen einer Genbank in z. B. der E. coli argE-Mutante bzw. in einer neu isolierten Mutante mit herkömmlichen Verfahren (Maniatis et al., Molecular Cloning: a Laboratory Manual, Cold Spring Harbor Laboratory, Cold Spring Harbor, New York, 1982) isolieren.

Verfahren zur Isolierung weiterer Deacetylasegene ergeben sich aus dem oben Beschriebenen: Z. B. Isolierung neuer Organismen, die trotz Anwesenheit eines pat-Gens auf einem Niedrigkopienzahlvektor PTT-sensitiv sind, und anschließende Isolierung eines Deacerylasegens.

pat- und dea-Gene können in einem weiteren Aspekt der Erfindung zusammen mit gewebespezifischen Promotoren eingesetzt werden, um die Entwicklung bestimmter Pflanzengewebe gezielt zu verhindern. Eine spezielle Anwendung ist z. B. die Herstellung männlich steriler Pflanzen.

Die Herstellung von Hybridsaatgut in der Pflanzenzüchtung ist davon abhängig, eine Selbstbefruchtung der Mutterpflanze mit großer Sicherheit zu vermeiden. In der Natur kommen für viele Pflanzenarten männlich sterile Mutanten vor, die in der Züchtung eingesetzt werden. Der molekulare Mechanismus der cytoplasmatischen männlichen Sterilität (cms) ist bis heute nicht vollständig geklärt Auch gibt es für viele Kultursorten, wie z. B. Beta vulgaris keine cms-Variante. Es ist daher von großem Interesse für die Landwirtschaft, auf molekulargenetischem Wege definierte cms-Mutanten aller wichtigen Kultursorten zu erzeugen. Die Firma PGS/Belgien hat in der Patentanmeldung PCT/EP 89/00495 eine solche Methode vorgestellt. Sie beruht auf der Zerstörung des die Pollenmutterzellen umgebenden Gewebes (Tapetum). Zu diesem Zweck wird ein RNAse-Gen mit einem Tapetum-spezifischen Promotor (Mariani et al.; Nature 347, 737-741, 1990) fusioniert. Die ausschließliche Expression des Gens in den Tapetumzellen sorgt für die selektive Zerstörung des Gewebes und verhindert damit die Bildung reifen Pollens. Eine Pflanze, die dieses Gen trägt, sollte nur nach Fremdbefruchtung Samen bilden können. Ein wesentlicher Nachteil dieses Systems ist die Tatsache, daß Nachkommen dieser Pflanze ebenfalls männlich steril sind und daher im Feld, wo sie auf Selbstbefruchtung angewiesen sind, keine Samen bilden können. Dies gelingt nur dann, wenn der männliche Partner der Kreuzung ein Gen trägt, das die Wirkung der RNAse in den Nachkommen aufheben kann. Dies soll nach der oben genannten offengelegten Patentanmeldung durch das barstar-Gen erfolgen. Tatsache hierbei ist, daß nur genetisch veränderte, das heißt transgene Partner in der Kreuzung genutzt werden können.

Nachstehend sind Verfahren zur Herstellung von cms-Pflanzen vorgestellt, die es zulassen, transgene Mutterpflanzen mit beliebigen artgleichen Partnern zu kreuzen. Dies wird durch Kombination eines dea-Gens unter Kontrolle z.B. eines Tapetum-Promotors in Verbindung mit einem konstitutiv exprimierten pat-Gen erreicht Durch Applikation von PTC bzw. PTT wird gezielt die Glutaminsynthethase in den Tapetumzellen gehemmt und diese zum Absterben gebracht. Ein noch einfacheres System besteht in der Herstellung transgener Pflanzen, die lediglich ein einziges Fremd-Gen enthalten, nämlich ein dea-Gen unter Kontrolle eines gewebespezifischen, hier Tapetum-Promotors, sowie Applikation von N-Ac-PTC bzw. N-Ac-PTT auf die Pflanze.

Verallgemeinert umfaßt die Erfindung folglich zur gewebespezifischen Inhibierung mit Hilfe eines Deacetylasegens, vorzugsweise vorgenannten dea-Gens aus E. coli oder S. viridochromogenes T0 494 folgende Verfahren:
1) Durch Pat-Aktivität PTT bzw. PTC-resistente Pflanzen (z. B. erzeugt wie in EP 0257542 beschrieben) werden mit dem Deacetylasegen aus Streptomyceten unter Kontrolle eines in Pflanzen gewebespezifischen Promotors transformiert. Nach Applikation von PTT oder PTC führt die Expression des Deacetylasegens zur Aufhebung der Phosphinothricin-N-Acetyltransferase-Aktivität in den entsprechenden Geweben. Diese werden dann selektiv abgetötet, während die restliche Pflanze resistent ist.
2) PTT- bzw. PTC-resistente Pflanzen werden mit dem E. coli Deacetylasegen unter Kontrolle eines gewebespezifischen Promotors transformiert. Nach Applikation von PTT oder PTC führt die Expression des Deacetylasegens zur Aufhebung der Phosphinothricin-N-Acetyltransferase-Aktivität in den entsprechenden Geweben. Diese werden dann selektiv abgetötet, während die restliche Pflanze resistent ist.
   Durch Verwendung von N-Acetyl-Phosphinothricin bzw. N-Acetyl-Phosphinothricin-Tripeptid kann dieses System vereinfacht werden. Beide Substanzen sind nicht als Herbizid aktiv, werden aber von Pflanzen aufgenommen, transportiert und nicht sofort abgebaut. Eine Deacetylaseaktivität für N-Acetyl-Phosphinothricin und N-Acetyl-Phosphinothricin-Tripeptid ist bisher in Pflanzen nicht nachgewiesen. So läßt sich das oben beschriebene 2-Gen-System auf ein 1-Gen-System reduzieren und damit entscheidend vereinfachen, wie unten weiter ausgeführt:
3) Beliebige Pflanzen werden mit einem Streptomyceten-Deacetylasegen unter Kontrolle eines gewebespezifischen Promotors transformiert. Nach Applikation von N-Acetyl-Phosphinothricin oder N-Acetyl-Phosphinothricin-Tripeptid führt die gewebespezifische Expression zum sofortigen Absterben des entsprechenden Gewebes.
4) Beliebige Pflanzen werden mit einem Deacetylasegen aus E.coli unter Kontrolle eines gewebespezifischen Promotors transformiert. Nach Applikation von N-Acetyl-Phosphinothricin oder N-Acetyl-Phosphinothricin-Tripeptid führt die gewebespezifische Expression zum sofortigen Absterben des entsprechenden Gewebes.

Wegen der höheren Spezifität der Streptomyceten-Deacetylase für N-Acetyl-Phosphinothricin-Tripeptid wird man im Fall 3) vorzugsweise N-Acetyl-Phosphinothricin-Tripeptid, im Fall 4) N-Acetyl-Phosphinothricin einsetzen, wenn man hohe Aktivitäten benötigt. Als gewebespezifische Promotoren können alle beschriebenen Promotoren Verwendung finden, deren selektive Expression in bestimmten Geweben nachgewiesen ist (z. B. Koltunow et al., The Plant Cell., Vol. 2, 1201-1224, 1990). Natürlich sind auch alle neu isolierten Promotoren mit ähnlichen Eigenschaften geeignet. Außer gewebespezifischen Promotoren können auch solche Promotoren eingesetzt werden, die einer anderen Art der Regulation (z. B. zeitlich, streßbedingt, umweltabhängig) unterworfen sind und die gewebespezifisch auftritt.

Diese Verfahren ermöglichen des weiteren die Analyse der Differenzierung der Zellregulation sowie die Erzeugung von Pflanzen, in denen die Entwicklung bestimmter Pflanzenteile gezielt verhindert wurde, vorzugsweise die Herstellung männlich steriler Pflanzen.

Weitere Aspekte der Erfindung sind in den Beispielen aufgeführt.

### Beispiel 1: Fusion des Deacetylasekodierbereichs mit eukaryontischen Transkriptionssignalen

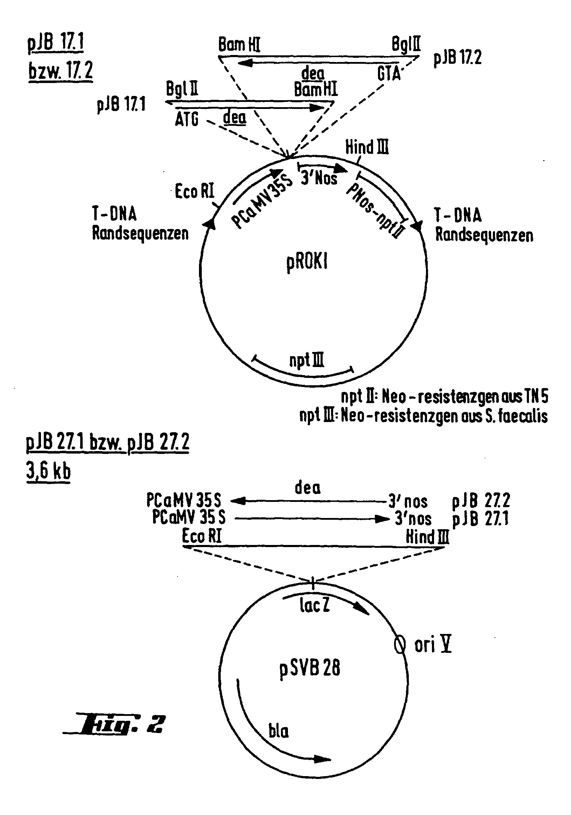

Aus einem E. coli Stamm wurde das Plasmid pPRI (siehe EP-0 257 542) isoliert und mit BamH1 und Bglll gespalten. Die verdaute DNA wurde in einem Agarosegel aufgetrennt und ein 0.9 kb Fragment aus dem Gel isoliert. Der Vektor pROKI (Baulcombe et al., Nature 321, 446-449, 1986) wurde ebenfalls mit BamHI restringiert. Die beiden Ansätze wurden vereinigt und ligiert. Das Ligationsgemisch wurde nach E. coli S17.1 (Simon et al., Bio/Technology 1, 784-791, 1983) transformiert. Auf kanamycinhaltigen Medien wachsende Kolonien wurden auf Nitrozellulosefilter übertragen und nach 12h Inkubation bei 37°C lysiert. Die DNA der Bakterien wurde auf dem Filter fixiert das aus dem Agarosegel isolierte 0,9kb Fragment wurde durch Inkubation bei 100°C einzelsträngig gemacht. Anschließend wurde der fehlende Strang mit Klenowpolymerase und Digoxigenin markierten Nukleotiden aufsynthetisiert. Der markierte Strang wurde als Probe zur Hybridisierung mit der auf den Filter gebundenen bakteriellen DNA genutzt. Hybridisierende Klone ließen sich mit Hilfe einer Antikörperreaktion nachweisen. Die DNA der positiven Klone wurde mittels Qiagen-Lyse isoliert und mit BarnHI/EcoRI sowie BamHI/HindIII verdaut. Diese Restriktion ermöglicht die Bestimmung der Orientierung des inserierten 0.9kb Fragmentes. Das Plasmid mit der Orientierung I wurde als pIB17.1, das mit der Orientierung II als p1B17.2 bezeichnet (siehe Fig. 2).

### Beispiel 2: Nachweis der Deacetylierung von N-Acetyl-PTC und N-Acetyl-PTT durch das Deacetylasegen

Es konnte gezeigt werden, daß die in dem Vektor pROKI klonierten eukaryontischen Transkriptionssignale auch eine Expression in R. meliloti. A. tumefaciens und E. coli ermöglichen.

Die Plasmide p1B17.1 und pIB17.2 wurden daher mittels 2-Faktor-Kreuzung in den Rhizobium meliloti Stamm 2011 transferriert. Durch Inkubation von R. meliloti Wildtypstämmen mit radioaktiv markiertem N-Acetyl-PTC konnte gezeigt werden, daß dieser Stamm N-Acetyl-PTC nicht deacetyliert (Nach Inkubation von pIB17.1 tragenden Stämmen mit N-Acetyl-PTC und N-Acetyl-PTT kann die Deacetylierung mittels Dünnschichtchromatographie nachgewiesen werden). Es konnte ebenfalls gezeigt werden, das R. meliloti sehr sensitiv auf PTC und PTT reagiert. Daher läßt sich die Deacetylierung auch über die Hemmung der R. meliloti Glutaminsynthetasen durch das freigesetzte PTC nachweisen.

### Beispiel 3: Transfer des modifizierten Deacetylasegens in Nicotiana tabacum

Das in Beispiel 1 modifizierte Deacetylasegen wurde mittels einer 2-Faktor Kreuzung nach A. tumefaciens LBA4404 transferiert. Mit den so entstandenen Stammen LBA4404117.1 und LBA4404/17.2 wurden Nicotiana tabacum Blattscheiben inkubiert und nach 3 Tagen auf ein Kanamycin haltiges Sprossinduktionsmedium umgesetzt Regenerierende kanamycinresistente Sprosse können durch Southern-hybridisierung auf die Anwesenheit des Deacetylasegens getestet werden. Nach Behandlung mit N-Aceiyl-PTC oder N-Acetyl-PTT werden dann die Pflanzen durch das freigesetzte PTC bzw. PTT abgetötet

### Beispiel 4: Konstruktion eines Vektors zur transienten Expression des modifizierten Deacetylasegens in E. coli und Tabakprotoplasten

Das modifizierte Deacetylasegen aus pIB17.1 und plB17.2 wurde durch EcoRI/HindIII Verdauung aus den Plasmiden herausgeschnitten. Die restringierte DNA wurde im Agarosegel aufgetrennt und jeweils ein 0,9 kb Fragment isoliert. Der Vektor pSVB28 (Arnold und Pühler, Gene 70, 171-179, 1988) wurde ebenfalls mit EcoRI/HindIII verdaut. Die beiden Ansätze wurden vereinigt und ligiert. Nach Transformation in den β-Galadosidase-negativen E. coli Stamm JM83 zeigten alle Vektor tragenden Klone eine Blaufärbung, während Klone, die einen Vektor mit Insertion des Deacetylasegens tragen, weiß blieben. Aus den so identifizierten Klonen wurde die DNA isoliert und mit EcoRI/HindIII verdaut. Anhand des Restriktionsmusters ließen sich die Klone mit dem modifizierten Deacetylasegen erkennen. Die konstruierten Vektoren tragen die Bezeichnung pIB27.1 und pIB27.2 (siehe Fig. 2). Sie liegen in E. coli mit großer Kopienzahl vor.

### Beispiel 5: Transiente Expression des modifizierten Deacetylasegens in Tabakprotoplasten

Aus den in Beispiel 4 konstruierten E. coli Stammen wurde die Plasmid-DNA isoliert. Junge Tabakblätter wurden mit Verdauungsenzymen für 20h inkubiert- Die aus dem Blattgerippe fallenden Protoplasten wurden gereinigt und in einem Transferpuffer mit Polyethylenglycol (PEG) und der isolierten DNA inkubiert. Anschließend wurden die Protoplasten gewaschen und in einer Kulturflüssigkeit (K3-Medium) aufgenommen. Nach 3 Tagen Inkubation bei schwacher Beleuchtung wurden die regenerierenden Protoplasten aufgeschlossen und die Rohextrakte mit radioaktiv markiertem N-Acetyl-PTC und N-Acetyl-PTT inkubiert Das deacetylierte PTC bzw. PTT läßt sich über Dünnschichtchromatographie nachweisen.

### Beispiel 6: Verfahren zur Erzeugung männlich steriler Kulturpflanzen unter Verwendung des Deacetylasegens aus S. viridochromogenes unter Kontrolle eines tapetumspezifischen Promotors.

Das Deacetylasegen aus Streptomyces viridochromogenes wird mit einem tapetumspezifischen Promotor aus Nicotiana tabacum fusioniert und über Agrobakterien vermittelte Blattscheiben-Transformation in Tabakzellen eingebracht Die aus diesen Zellen regenerierenden Pflanzen werden zu einem beliebigen Zeitpunkt vor der Blüte mit N-Acetyl-PTC oder N-Acetyl-PTT gespritzt. Es kann gezeigt werden, daß N-Acetyl-PTC in der Pflanzenzelle stabil ist und in alle Zellen transportiert wird. Keine der beiden Substanzen bat erkennbare negative Folgen für die Wildtyppflanze. Sobald sich die ersten Tapetumzellen bilden, beginnen sie mit der Expression des Deacetylasegens. Das in der Zelle gespeicherte N-Acetyl-PTC oder N-Acetyl-PTT wird durch das Enzym deacetyliert und damit in seine wirksame Form überführt. Es hemmt die Glutaminsynthetase der Zellen und führt so zu einem schnellen Absterben. Reife Pollen können nicht mehr entstehen. Zusätzlich ist auch die Bildung der Deacetylase unterbrochen. Umliegende Zellen sollten nicht beeinträchtigt werden. Wird die Pflanze nicht mit N-Acetyl-PTC oder N-Acetyl-PTT behandelt, ist sie voll fertil. Damit erübrigt sich eine Aufhebung der cms durch ein Gen des männlichen Partners der Kreuzung. Gleichzeitig liegt eine genau definierte Mutation vor, die ohne Auswirkungen auf die Wüchsigkeit und Nutzbarkeit der Pflanze bleibt

## Patentansprüche

1. Pflanze, **dadurch gekennzeichnet, dass** sie ein Deacetylasegen, welches für eine N-Actetyl-PTC- oder N-Acetyl-PTT-Deacetylase kodiert, unter der Kontrolle eines gewebespezifischen Promotors enthält, und wobei die Expression des Deacetylasegens unter der Kontrolle des gewebespezifischen Promotors in bestimmten Geweben dieser Pflanze nach Behandlung mit N-Acetyl-PTC oder N-Acetyl-PTT und die Umsetzung von N-Acetyl-PTC oder N-Acetyl-PTT zu PTC oder PTT zum Absterben der betreffenden Gewebeteile führt.

2. Pflanze gemäß Anspruch 1, **dadurch gekennzeichnet, dass** sie ein Phosphinothricin-N-Acetyltransferase-Gen enthält, welches für eine Phosphinothricin-N-Acetyl-Transferase kodiert, und wobei die Behandlung der Pflanze mit PTC oder PTT und deren Umsetzung zu N-Acetyl-PTC oder N-Acetyl-PTT durch die Phosphinothricin-N-Acetyltransferase in der gesamten Pflanze, sowie die gewebespezifische Deacetylierung von N-Acetyl-PTC oder N-Acetyl-PTT zu PTC oder PTT durch die N-Acetyl-PTC- oder N-Acetyl-PTT-Deacetylase in den betreffenden Gewebeteilen zum Absterben der betreffenden Gewebeteile führt.

3. Pflanze gemäß einem der Ansprüche 1 oder 2, **dadurch gekennzeichnet, dass** diese Pflanze eine männlich sterile Pflanze ist.

4. Pflanze gemäß einem der Ansprüche 1 bis 3, wobei das Deacetylasegen unter Kontrolle eines Tapetum-Promotors steht.

5. Pflanze gemäß einem der Ansprüche 1 bis 4, wobei das Deacetylasegen aus *Escherichia coli* stammt.

6. Pflanze gemäß einem der Ansprüche 1 bis 4, wobei das Deacetylasegen aus *Streptomyces viridochromogenes* Tü494 stammt.

7. Pflanze gemäß Anspruch 6, wobei die Proteinsequenz der Deacetylase durch das offene Leseraster der DNA-Sequenz gemäß Tabelle 1 kodiert ist.

8. Pflanze gemäß Anspruch 6, wobei das Deacetylasegen das offene Leseraster der Nukleotidsequenz gemäß Tabelle 1 enthält.

## Claims

1. A plant which comprises a deacetylase gene coding for an N-acetyl-PTC- or N-acetyl-PTT-deacetylase under the control of a tissue-specific promoter and where the expression of the deacetylase gene under the control of the tissue-specific promoter in certain tissues of this plant after treatment with N-acetyl-PTC or N-acetyl-PTT and the conversion of N-acetyl-PTC or N-acetyl-PTT to PTC or PTT leads to the dying of the corresponding tissues.

2. The plant as claimed in claim 1, which comprises a phosphinothricin-N-acetyltransferase gene coding for a phosphinothricin-N-acetyltransferase and where the treatment of the plant with PTC or PTT and their conversion to N-acetyl-PTC or N-acetyl-PTT by the phosphinothricin-N-acetyltransferase in the entire plant, and the tissue-specific deacetylation of N-acetyl-PTC or N-acetyl-PTT to PTC or PTT by the N-acetyl-PTC- or N-acetyl-PTT-deacetylase in the corresponding tissues leads to the dying of the corresponding tissues.

3. The plant as claimed in claim 1 or 2, which is a male-sterile plant.

4. The plant as claimed in any of claims 1 to 3, wherein the deacetylase gene is under the control of a tapetum promoter.

5. The plant as claimed in any of claims 1 to 4, wherein the deacetylase gene is derived from *Escherichia coli.*

6. The plant as claimed in any of claims 1 to 4, wherein the deacetylase gene is derived from *Streptomyces viridochromogenes* Tü494.

7. The plant as claimed in claim 6, wherein the protein sequence of the deacetylase is encoded by the open reading frame of the DNA sequence as shown in Table 1.

8. The plant as claimed in claim 6, wherein the deacetylase gene comprises the open reading frame of the nucleotide sequence as shown in Table 1.

## Revendications

1. Plante, **caractérisée en ce qu'**elle contient un gène de déacétylase, qui code pour une N-acétyl-PTC- ou N-acétyl-PTT-déacetylase, sous le contrôle d'un promoteur spécifique de tissu, et l'expression du gène de déacétylase sous le contrôle du promoteur spécifique de tissu conduisant dans certains tissus de cette plante, après traitement par la N-acétyl-PTC ou N-acétyl-PTT et transformation de la N-acétyl-PTC ou N-acétyl-PTT en PTC ou PTT, à la mort des parties de tissu concernées.

2. Plante selon la revendication 1, **caractérisée en ce qu'**elle contient un gène de phosphinothricine-N-acétyltransférase, qui code pour une phosphinothricine-N-acétyl-transférase, et le traitement de la plante par la PTC ou PTT et leur transformation en N-acétyl-PTC ou N-acétyl-PTT par la phosphinothricine-N-acétyltransférase dans l'ensemble de la plante, ainsi que la déacétylation spécifique de tissu de la N-acétyl-PTC ou N-acétyl-PTT en PTC ou PTT par la N-acétyl-PTC- ou N-acétyl-PTT-déacétylase dans les parties de tissus concernées conduisant à la mort des parties de tissu concernées.

3. Plante selon l'une des revendications 1 ou 2, **caractérisée en ce que** cette plante est une plante à stérilité masculine.

4. Plante selon l'une des revendications 1 ou 3, le gène de déacétylase étant sous le contrôle d'un promoteur spécifique du tapetum.

5. Plante selon l'une des revendications 1 à 4, le gène de la déacétylase provenant de l*'Escherichia coli.*

6. Plante selon l'une des revendications 1 à 4, le gène de la déacétylase provenant du *Streptomyces viridochromogenes* T494.

7. Plante selon la revendication 6, la séquence de la protéine de la déacétylase étant codée par le cadre de lecture ouvert de la séquence d'ADN selon le Tableau 1.

8. Plante selon la revendication 6, le gène de la déacétylase contenant le cadre de lecture ouvert de la séquence nucléotidique selon le Tableau 1.


### SEQUENZPROTOKOLL

(1) ALGEMEINE INFORMATION:
   (i) ANMELDER:
      (A) NAME: Hoechst Schering AgrEvo GmbH
      (B) STRASSE: Gebaeude K 801
      (C) ORT: Frankfurt
      (D) BUNDESLAND: -
      (E) LAND: Germany
      (F) POSTLEITZAHL: 65926
      (G) TELEPHON: 069-305-82808
      (H) TELEFAX: 69-305-2200
      (I) TELEX: -
   (ii) ANMELDETITEL: Deacetylasegen zur Erzeugung von Phosphinothricin oder Phosphinothricyl-Alanyl-Alanin, Verfahren zu ihrer Isolierung und ihre Verwendung
   (iii) ANZAHL DER SEQUENZEN: 2
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1a:
   (i) SEQUENZ CHARAKTERISTIKA:
      (A) LÄNGE: 932 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..932
   (xi) SEQUENZBESCHREIBUNG: SEQ ID NO: 1a: 
AGATCTGAGC GGAGAGCGCA TGGCATCGTC GGAGTTGGAG CTCGTGCGGG AACTGATCGG 60
GCTCAACTGG CACACCCGCA ACGGCGATGT GGAGCCACGC CGGGTGGCCT ACGACCGAGC 120
CCAGGAGGCC TTCGGGCACC TGGGCCTGCC CCCCGGCGAG ACCGTCGTGA TCGGCGACTG 180
CTCGGCGGAG TGGGTACGGC CCGCCCAGGA GGACGGCAGG ACCCTGCTGT ACCTGCACGG 240
CGGTTCGTAC GCCCTCGGAT CGCCGCAGTC GCACCGCCAT CTGTCCAGCG CGCTGGGCGC 300
GGCGGCCGGG GCGGCGGTGC TCGCCCTGCA CTACCGCAGG CCGCCCGAGT CTCCCTTCCC 360
GGCGGCGGTG GAGGACGCCG TGGCGGCCTA CCGGATGCTG CGGGAGCGGG GCCTGCCGCC 420
GGGGCGGATC ACCTTCGCCG GTGACTCGGC CGGCGCGGGC CTCGCCGTCG CCGCCCTCCA 480
GGTGCTGCGC GACGCCGGGG ACCCGCTGCC GGCCGCCGCG GTGTGCATCT CGCCCTGGGC 540
CGACCTGGCC TGCGAGGGCG CCTCGCACGT CACCCGCAAG GAGCGCGAGA TCCTCCTGGA 600
CACCGAGGAC CTGCTCCGCA TGGCGGGGCG CTACCTGGCC GGCACCGATC CCAGGAACCC 660
CCTGGCCTCG CCCGCCCACG GCGATCTGAC CGGTCTGCCC CCCCTGCTCA TCCAGGTCGG 720
 
TTCCGAGGAA GTCCTGTACG ACGACCCCCG GGCGCTGGAA CAGGCGGCGC TCAAGGCGGG 780

CGTACCGGTC ACCTTCGACG AGTGGCCGGA GATGTTCCAC GTCTGGCACT GGTACCACCC 840

GGTGCTCCCC GAGGGGCGTG CCGCCGTCGA GACGGCGGGC GTGTTCCTGC GCCGCGCCAC 900

CGAGGAGGGC GAGCGGTGAC CGACTGGATC CT 932
(2) INFORMATION ZU SEQ ID NO: 1b:
   (i) SEQUENZ CHARAKTERISTIKA:
      (A) LÄNGE: 299 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..299
   (xi) SEQUENZBESCHREIBUNG: SEQ ID NO: 1b: